(19) Europäisches Patentamt
European Patent Office
Office européen des brevets

(11) EP 0 459 819 B1

(12) **EUROPEAN PATENT SPECIFICATION**

(45) Date of publication and mention of the grant of the patent:
**14.08.1996 Bulletin 1996/33**

(51) Int. Cl.[6]: **C07D 239/48**, A61K 31/505

(21) Application number: **91304935.9**

(22) Date of filing: **31.05.1991**

(54) **Pharmacologically active CNS compound**

Pharmazeutisch wirksame ZNS-Verbindung

Composé pharmaceutiquement actif sur le SNC

(84) Designated Contracting States:
**AT BE CH DE DK ES FR GB GR IT LI LU NL SE**

(30) Priority: **01.06.1990 GB 9012316**

(43) Date of publication of application:
**04.12.1991 Bulletin 1991/49**

(60) Divisional application: **95111314.1**

(73) Proprietor: **THE WELLCOME FOUNDATION LIMITED**
**Greenford, Middlesex UB6 0NN (GB)**

(72) Inventors:
- **Leach, Michael John**
  **Beckenham, Kent, BR3 3BS (GB)**
- **Nobbs, Malcolm Stuart**
  **Beckenham, Kent, BR3 3BS (GB)**
- **Iyer, Ramachandran**
  **Beckenham, Kent, BR3 3BS (GB)**
- **Yeates, Clive Leonard**
  **Beckenham, Kent, BR3 3BS (GB)**
- **Skone, Philip Alan**
  **Beckenham, Kent, BR3 3BS (GB)**

(74) Representative: **Woods, Geoffrey Corlett et al**
**J.A. KEMP & CO.**
**14 South Square**
**Gray's Inn**
**London WC1R 5LX (GB)**

(56) References cited:
**EP-A- 0 372 934** **BE-A- 518 622**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 459 819 B1

Printed by Rank Xerox (UK) Business Services
2.13.0/3.4

## Description

The present invention relates to a 5-aryl pyrimidine, to processes for its preparation, to pharmaceutical formulations containing it and to its use in therapy, in particular the treatment of central nervous system (CNS) diseases and disorders.

Glutamate is an excitatory amino acid which functions as a neurotransmitter. However, when its extracellular concentration is sufficiently high, glutamate acts as a powerful neurotoxin, capable of killing neurones in the central nervous system (Rothman & Olney (1986) Prog. Brain. Res., 63, 69). The neurotoxic effect of glutamate has been implicated in a number of central nervous system disorders and disease states including cerebral ischaemic damage and chronic neurodegenerative disorders such as Alzheimer's disease, motor system disorders and Huntington's chorea (Meldrum Clinical Science (1985) 68 113-122). In addition, glutamate has been implicated in other neurological disorders such as epilepsy, manic depression, depression, schizophrenia, high pressure neurological syndrome, chronic pain, trigeminal neuralgia and migraine.

European Patent Application No. 89312723.3 (EP-A-0372934) discloses a generic class of substituted phenylpyrimidines for use in the manufacture of a medicament for treating or preventing CNS disorders or diseases of a mammal.

The present invention provides 4-amino-2-(4-n-propylpiperazin-1-yl)-5-(2,3,5-trichlorophenyl)pyrimidine and acid addition salts thereof. 4-Amino-2-(4-n-propylpiperazin-1-yl)-5-(2,3,5-trichlorophenyl)pyrimidine is a potent inhibitor of extracellular glutamate release and has a negligible effect against the enzyme dihydrofolate reductase.

Suitable acid addition salts include those formed with both organic and inorganic acids. Such acid addition salts will normally be pharmaceutically acceptable although salts with non-pharmaceutically acceptable acids may be of utility in the preparation and purification of the pyrimidine of the invention. Thus, preferred salts include those formed from hydrochloric, hydrobromic, sulphuric, citric, tartaric, phosphoric, lactic, pyruvic, acetic, succinic, fumaric, maleic, oxaloacetic, methanesulphonic, ethanesulphonic, p-toluenesulphonic, benzenesulphonic and isethionic acids. Salts can be made by reacting the pyrimidine of the invention in the form of the free base with the appropriate acid.

The pyrimidine of the invention and pharmaceutically acceptable acid addition salts thereof may be used for the treatment or prophylaxis in a mammal (including man) of a neurodegenerative or other neurological disorder of the central nervous system, the aetiology of which includes excessive release of neurotransmitter glutamate.

Such disorders can be either acute or chronic. Acute conditions include cerebral ischaemic damage which may arise from a variety of causes including stroke, cardiac arrest, cardiac bypass surgery, neonatal anoxia and hypoglycaemia; and also physical injury or trauma of the spinal cord or brain. Chronic disorders include Alzheimer's disease, Huntington's chorea, olivopontocerebellar atrophy and motor system disorders. Other neurological conditions which may be treated with a compound of the invention include depression, manic depression, schizophrenia, chronic pain, epilepsy, trigeminal neuralgia and migraine.

While it is possible for the pyrimidine of the present invention to be administered as the raw chemical, it is preferable to present it as a pharmaceutical formulation. A pharmaceutical formulation of the present invention comprises 4-amino-2-(4-n-propylpiperazin-1-yl)-5-(2,3,5-trichlorophenyl)pyrimidine or a pharmaceutically acceptable acid addition salt thereof together with one or more pharmaceutically acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy. All methods include the step of bringing into association 4-amino-2-(4-n-propylpiperazin-1-yl)-5-(2,3,5-trichlorophenyl)pyrimidine or a pharmaceutically acceptable acid addition salt thereof ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

Preferred unit dosage formulations are those containing an effective dose, as hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

Tablets or other forms of presentation provided in discrete units may conveniently contain an amount of 4-amino-2-(4-n-propylpiperazin-1-yl)-5-(2,3,5-trichlorophenyl)pyrimidine which is effective at such dosage or as a multiple of the same, for instance, units containing 5 mg to 500 mg, usually around 10 mg to 250 mg.

The 4-amino-2-(4-n-propylpiperazin-1-yl)-5-(2,3,5-trichlorophenyl)pyrimidine is preferably used to treat CNS disorders or diseases by oral administration or injection (intraparenteral or subcutaneous). The precise amount of compound administered to a patient will be the responsibility of the attendant physician. However the dose employed will depend on a number of factors, including the age and sex of the patient, the precise disorder being treated, and its severity. Thus for example when treating a patient with epilepsy the dose range is likely to be significantly lower than when treating a patient after stroke to alleviate cerebral ischaemic damage. Also the route of administration is likely to vary depending on the condition and its severity.

The 4-amino-2-(4-n-propylpiperazin-1-yl)-5-(2,3,5-trichlorophenyl)pyrimidine may be administered orally or via injection at a dose of from 0.1 to 30 mg/kg per day. The dose range for adult human beings is generally from 8 to 2,400 mg/day and preferably 35 to 1,050 mg/day.

The present invention provides a first process for the preparation of 4-amino-2-(4-n-propylpiperazin-1-yl)-5-(2,3,5-trichlorophenyl)pyrimidine or an acid addition salt thereof, which process comprises reacting a compound of formula (I):

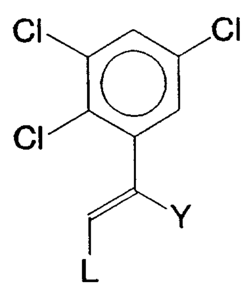

(I)

wherein L is a leaving group and Y is cyano, with the compound of formula (II):

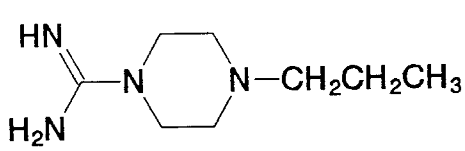

(II)

or a salt thereof; and isolating the thus formed 4-amino-2-(4-n-propylpiperazin-1-yl)-5-(2,3,5-trichlorophenyl)pyrimidine as the free base or as an acid addition salt thereof.

Examples of suitable leaving groups include alkoxy e.g. $C_{1-4}$ alkoxy, alkylthio e.g. $C_{1-4}$ alkylthio, halo and optionally substituted amino, e.g. a group of formula $NR^9R^{10}$ where $R^9$ and $R^{10}$ are the same or different and are selected from hydrogen, alkyl, aryl and arylalkyl or $R^9$ and $R^{10}$, together with the nitrogen atom to which they are attached, form a

heterocyclic ring optionally substituted by one or more alkyl groups and optionally containing a further heteroatom. The group $NR^9R^{10}$ may therefore be anilino, morpholino, $C_{1-4}$ alkylamino or benzylamino.

Preferably the reaction of the compounds of formulae (I) and (II) is carried out in a non-aqueous solvent, for example an alkanol such as ethanol, at elevated temperatures (e.g. from 50 to 110°C). The compound of formula (II) is generally used in the form of a salt such as the hydroiodide salt, in which case the reaction is preferably carried out in the presence of a base, such as an alkanoxide, for example sodium ethoxide, preferably under reflux. Where the compound of formula (II) is used in the form of the free base, the addition of further base may be unnecessary.

The invention additionally provides a second process for the preparation of 4-amino-2-(4-n-propylpiperazin-1-yl)-5-(2,3,5-trichlorophenyl)pyrimidine or an acid addition salt thereof, which process comprises reacting a compound of formula (III):

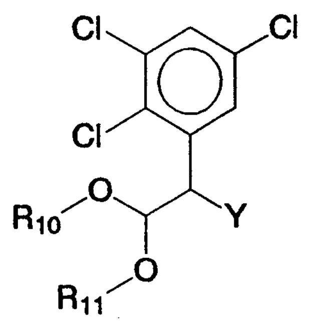

(III)

wherein Y is cyano and $R_{10}$ and $R_{11}$ are both alkyl or together form a group $-(C(R)_2)_n-$ where R is hydrogen or alkyl and n is an integer from 2 to 4, with the compound of formula (II) defined above or a salt thereof; and isolating the thus formed 4-amino-2-(4-n-propylpiperazin-1-yl)-5-(2,3,5-trichlorophenyl)pyrimidine as the free base or as an acid addition salt thereof.

The reaction may be carried out in a non-aqueous solvent, eg. ethanol, under reflux. The compound of formula (II) is generally used in the form of a salt such as the hydroiodide salt, in which case the reaction is preferably carried out in the presence of a base such as an alkanoxide, for example sodium ethoxide. Where the compound of formula (II) is used in the form of the free base, the addition of further base may be unnecessary.

The pyrimidine of the invention can be isolated as the free base or as an acid addition salt. The pyrimidine in the form of the free base can be converted into an acid addition salt by standard methods, for example by reacting the free base with the appropriate acid in a suitable solvent. Similarly one acid addition salt can be converted into another acid addition salt by standard methods.

Compounds of formula (I) can be made by known methods (see for example J. Amer. Chem. Soc. 1951, 73, 3763-3770) for example by the reaction of 2-(2,3,5-trichlorophenyl)-3-oxopropionitrile with diazomethane or with alkylorthoesters (J. Amer. Chem. Soc., 1952, 74, 1310-1313) or by condensation with an amine. 2-(2,3,5-Trichlorophenyl)-3-oxopropionitrile may be made by known methods (J. Amer. Chem. Soc., 1951, 73, 3763-3770).

The compound of formula (II) can also be made by standard methods, for example by reaction of a compound of formula $H_2N-C(NH)-R^1$ in which $R^1$ is alkylthio with the appropriate amine. The reaction is preferably carried out at room temperature in a suitable solvent such as water.

The following Example illustrates the invention. Two Reference Examples are provided.

REFERENCE EXAMPLE 1 : 2-(2,3,5-trichlorophenyl)-3-methoxyacrylonitrile

(i) 2,3,5-Trichlorobenzylalcohol

To a solution of 2,3,5-trichlorobenzaldehyde (Aldrich, 50 gms) in ethanol (1.0 l) at room temperature was added $NaBH_4$ (7.00 gms) and the resulting mixture stirred for 3.5 hours. The reaction was quenched with water and the solvent evaporated in vacuo before partitioning the residue between $CHCl_3$ and saturated $NaHCO_3$ solution. The organic phase was washed with brine, dried over $MgSO_4$, filtered and the solvent evaporated in vacuo to leave a white solid, 43.00 gms, mp. 90-93°C.

(ii) 2,3,5-Trichlorobenzyl bromide

To a solution of the alcohol in benzene (400 ml) under $N_2$ was added $PBr_3$ (126.58 gms) and the mixture stirred at 55-60°C for 3.5 hours. After cooling, the mixture was poured onto crushed ice (2 l) and the benzene layer separated. The aqueous phase was washed with benzene (x3) and the combined benzene extracts washed with saturated $NaHCO_3$ solution and water, dried over $MgSO_4$, filtered and the solvent evaporated to leave a brownish liquid which solidified on standing, 37.53 gms, mp. 40-42°C.

(iii) 2,3,5-Trichlorophenylacetonitrile

The bromide was suspended in dimethylformamide (DMF) (130 ml)/water (86.67 ml) at 0°C and KCN (12.99 gms) added in portions. After stirring at 30-35°C for 3 hours, the suspension was diluted with water and extracted with diethyl ether ($Et_2O$). The combined ether extracts were washed with water, dried over $MgSO_4$, filtered and the solvent evaporated in vacuo. Chromatography on silica gel eluting with hexane to 20% ether-hexane gave the desired product as a white solid, 18.52 gms, mp. 60-62°C.

(iv) 2-(2,3,5-Trichlorophenyl)-3-oxopropionitrile sodium salt

To a solution of sodium ethoxide (NaOEt)(from 0.803 g of sodium) in ethanol (55 ml) cooled in ice, under nitrogen, was added 2,3,5-trichlorophenylacetonitrile. Ethyl formate (5.1 ml) was added and the mixture was stirred at room temperature overnight. After stirring for a further 2.5 hours at 50°C, the mixture was cooled and filtered. The filtrate was evaporated, and the residue was triturated with diethyl ether, filtered and dried (6.82 g).

(v) 2-(2,3,5-Trichlorophenyl)-3-methoxyacrylonitrile

The above solid was dissolved in DMF (36 ml) and methyl iodide (2 ml) was added. The reaction vessel was sealed before stirring the contents at 40°C for 3 hours. The solvent was then evaporated. The residue was partitioned between water and ethyl acetate. The organic phase was washed with water, dried ($MgSO_4$) and the solvent evaporated to give the crude product as a red-brown oil that solidified on standing (5.04 g).

REFERENCE EXAMPLE 2 : methylthiouronium iodide

Thiourea (10.8 g) was dissolved in acetone (250 ml) at 50°C. Iodomethane (10 ml) was added and the reaction was stirred at 50°C for 4 hours. After cooling, the solution was diluted with ether (1 litre) and the methiodide salt was filtered, washed with ether and dried in vacuo, 29.2 g, mp. 113-115°C.

EXAMPLE : 4-amino-2-(4-n-propylpiperazin-1-yl)-5-(2,3,5-trichlorophenyl)pyrimidine dimesylate

N-Propylpiperazine dihydrobromide (Lancaster 5g) in water (15ml) was passed into an ion exchange column (IR410 OH form) (BDH) and eluted with water. The eluate that was positive for secondary amine was concentrated, dissolved in diethylether, dried ($MgSO_4$) and evaporated to dryness to give n-propylpiperazine as a colourless oil (0.6g).

The piperazine was dissolved in water (10ml) and methylthiouronium iodide (Reference Example 2) added. The solution was stirred, with nitrogen bubbled through, at room temperature for 96 hours. The solution was concentrated in vacuo. The residue was slurried with acetone, filtered and dried in vacuo to give N-propylpiperazinoformamidine hydroiodide, 0.51g, mp. 174-176°C.

To a solution of NaOEt (from 0.52g of sodium) in ethanol (5ml) was added N-propylpiperazinoformamidine hydroiodide (0.506g). After stirring for a further 10 minutes, the 2-(2,3,5-trichlorophenyl)-3-methoxyacrylonitrile of Reference Example 1 (0.226g) was added and the mixture was stirred at reflux for 5 hours. The mixture was concentrated, dissolved in chloroform and filtered. The filtrate was reconcentrated and the residue was purified by chromatography on $SiO_2$, eluting with 5% $MeOH/CHCl_3$ to give the title compound as the free base (0.26g).

The free base (0.26g) was dissolved in $Et_2O$ and cooled in an ice bath. Methanesulphonic acid (0.062g) was added and the reaction was stirred in the ice bath for 1 hour. The solid was filtered and dried in vacuo to give the title salt as a yellow amorphous solid, 0.21g, dec. 83°C.

NMR Assignment (δ)

Solvent - DMSO

δ 0.95(t,3H), δ 1.75(m,2H), δ 2.35(s,6H), δ 3.10(d,br,2H), δ 3.40(t,br,4H), δ 3.60(d,br,2H), δ 4.60(d,2H), δ 7.45(d,1H), δ 7.90(s,1H), δ 7.95(d,1H)

Pharmacological Activity

Inhibition of Glutamate release and Inhibition of Rat Liver DHFR

4-Amino-2-(4-n-propylpiperazin-1-yl)-5-(2,3,5-trichlorophenyl)pyrimidine was tested for its effect on veratrine-evoked release of glutamate from rat brain slices according to the protocol described in Epilepsia 27(5): 490-497, 1986. The protocol for testing for inhibition of DHFR activity was a modification of that set out in Biochemical Pharmacology Vol. 20 pp 561-574, 1971. The results are given in Table 1, the $IC_{50}$ being the concentration of compound to cause 50% inhibition of (a) veratrine-evoked release of glutamate and (b) DHFR enzyme activity.

TABLE 1

| $IC_{50}$(μM) Glutamate release (P95 limits) | $IC_{50}$(μM) Rat Liver DHFR |
|---|---|
| 2.17 (1.5-2.9) | > 100 |

Pharmaceutical Formulation Examples

A. Injection

The salt of 4-amino-2-(4-n-propylpiperazin-1-yl)-5-(2,3,5-trichlorophenyl)pyrimidine was dissolved in sterile water for injection.

In the following pharmaceutical Formulation Examples B to D, the active ingredient may be 4-amino-2-(4-n-propylpiperazin-1-yl)-5-(2,3,5-trichlorophenyl)pyrimidine or a pharmaceutically acceptable acid addition salt thereof.

B. Capsule formulations

Capsule Formulation I

Formulation I may be prepared by admixing the following ingredients and filling two-part hard gelatin capsules with the resulting mixture:

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Capsule Formulation II

| | mg/capsule |
|---|---|
| (a) Active ingredient | 250 |
| (b) Macrogel 4000 BP | 350 |
| | 600 |

Capsules may be prepared by melting the Macrogel 4000 BP, dispersing the active ingredient in the melt, and filling two-part hard gelatin capsules therewith.

Capsule Formulation III (Controlled release capsule)

| | mg/capsule |
|---|---|
| (a) Active ingredient | 250 |
| (b) Microcrystalline cellulose | 125 |
| (c) Lactose BP | 125 |
| (d) Ethyl Cellulose | 13 |
| | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

C. Syrup formulation

| | |
|---|---|
| Active ingredient | 0.2500 g |
| Sorbitol Solution | 1.5000 g |
| Glycerol | 1.0000 g |
| Sodium Benzoate | 0.0050 g |
| Flavour | 0.0125 ml |
| Purified Water q.s. to | 5.0 ml |

The sodium benzoate is dissolved in a portion of the purified water and the sorbitol solution added. The active ingredient is added and dissolved. The resulting solution is mixed with the glycerol and then made up to the required volume with the purified water.

D. Suppository formulation

| | mg/suppository |
|---|---|
| Active ingredient (63 μm)* | 250 |
| Hard Fat, BP (Witepsol H15 - Dynamit Nobel) | 1770 |
| | 2020 |

* The active ingredient is used as a powder wherein at least 90% of the particles are of 63 μm diameter or less.

One-fifth of the Witepsol H15 is melted in a steam-jacketed pan at 45°C maximum. The active ingredient is sifted through a 200 μm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250μm stainless steel screen and, with continuous stirring, allowed to cool to 40°C. At a temperature of 38-40°C, 2.02g aliquots of the mixture are filled into suitable plastic moulds and the suppositories are allowed to cool to room temperature.

## Claims

**Claims for the following Contracting States : AT, BE, CH, DE, DK, FR, GB, IT, LI, LU, NL, SE**

1. 4-Amino-2-(4-n-propylpiperazin-1-yl)-5-(2,3,5-trichlorophenyl)pyrimidine and acid addition salts thereof.

2. A salt according to claim 1 which is a pharmaceutically acceptable acid addition salt.

3. A salt according to claim 1, which is a salt formed from hydrochloric, hydrobromic, sulphuric, citric, tartaric, phosphoric, lactic, pyruvic, acetic, succinic, fumaric, maleic, oxaloacetic, methanesulphonic, ethanesulphonic, p-toluenesulphonic, benzenesulphonic or isethionic acid.

4. A salt according to claim 1, which is 4-amino-2-(4-n-propylpiperazin-1-yl)-5-(2,3,5-trichlorophenyl)pyrimidine dimesylate.

5. A process for the preparation of 4-amino-2-(4-n-propylpiperazin-1-yl)-5-(2,3,5-trichlorophenyl)pyrimidine or an acid addition salt thereof, which process comprises reacting a compound of formula (I):

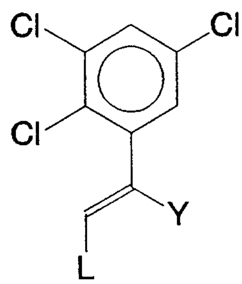

(I)

wherein L is a leaving group and Y is cyano, with the compound of formula (II):

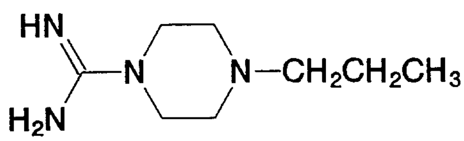

(II)

or a salt thereof; and isolating the thus formed 4-amino-2-(4-n-propylpiperazin-1-yl)-5-(2,3,5-trichlorophenyl)pyrimidine as the free base or as an acid addition salt thereof.

6. A process according to claim 5, wherein L is $C_{1-4}$ alkoxy.

7. A process for the preparation of 4-amino-2-(4-n-propylpiperazin-1-yl)-5-(2,3,5-trichlorophenyl)pyrimidine or an acid addition salt thereof, which process comprises reacting a compound of formula (III):

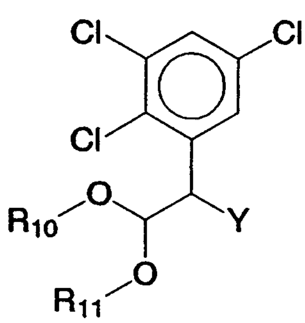

(III)

wherein Y is cyano and $R_{10}$ and $R_{11}$ are both alkyl or together form a group $-(C(R)_2)_n-$ where R is hydrogen or alkyl

and n is an integer from 2 to 4, with the compound of formula (II) defined in claim 5 or a salt thereof; and isolating the thus formed 4-amino-2-(4-n-propylpiperazin-1-yl)-5-(2,3,5-trichlorophenyl)pyrimidine as the free base or as an acid addition salt thereof.

**8.** A pharmaceutical formulation comprising 4-amino-2-(4-n-propylpiperazin-1-yl)-5-(2,3,5-trichlorophenyl)pyrimidine or a pharmaceutically acceptable acid addition salt thereof and one or more pharmaceutically acceptable carriers therefor.

**9.** A pharmaceutical formulation according to claim 8 which is suitable for oral administration.

**10.** A pharmaceutical formulation according to claim 9 which is in the form of capsules or tablets each containing a predetermined amount of 4-amino-2-(4-n-propylpiperazin-1-yl)-5-(2,3,5-trichlorophenyl)pyrimidine or a pharmaceutically acceptable acid addition salt thereof.

**11.** A pharmaceutical formulation according to claim 8 which is suitable for parenteral administration.

**12.** A pharmaceutical formulation according to claim 11 which is presented in unit-dose containers.

**13.** 4-Amino-2-(4-n-propylpiperazin-1-yl)-5-(2,3,5-trichlorophenyl)pyrimidine or a pharmaceutically acceptable acid addition salt thereof for use in the treatment or prophylaxis in a mammal of a neurodegenerative or other neurological disorder of the central nervous system, the aetiology of which includes excessive release of neurotransmitter glutamate.

**14.** A compound according to claim 13, wherein the disorder is cerebral ischaemic damage.

**15.** A compound according to claim 14, wherein the damage has been caused by stroke.

**16.** A compound according to claim 13, wherein the disorder is physical injury or trauma of the spinal cord or brain.

**Claims for the following Contracting States : ES, GR**

**1.** A process for the preparation of 4-amino-2-(4-n-propylpiperazin-1-yl)-5-(2,3,5-trichlorophenyl)pyrimidine or an acid addition salt thereof, which process comprises reacting a compound of formula (I):

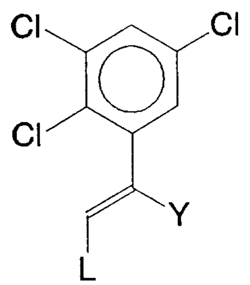

(I)

wherein L is a leaving group and Y is cyano, with the compound of formula (II):

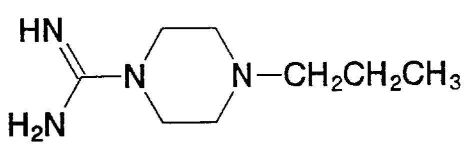

(II)

or a salt thereof; and isolating the thus formed 4-amino-2-(4-n-propylpiperazin-1-yl)-5-(2,3,5-trichlorophenyl)pyrimidine as the free base or as an acid addition salt thereof.

**2.** A process according to claim 1, wherein the said reaction is carried out in a non-aqueous solvent at from 50 to 110°C.

3. A process according to claim 2, wherein the solvent is ethanol.

4. A process according to any one of the preceding claims, wherein the compound of formula (II) is used in the form of the hydroiodide salt and the said reaction is carried out under reflux in the presence of a base.

5. A process according to claim 4, wherein the base is sodium ethoxide.

6. A process according to any one of the preceding claims, wherein the 4-amino-2-(4-n-propylpiperazin-1-yl)-5-(2,3,5-trichlorophenyl)pyrimidine is isolated as a salt formed from hydrochloric, hydrobromic, sulphuric, citric, tartaric, phosphoric, lactic, pyruvic, acetic, succinic, fumaric, maleic, oxaloacetic, methanesulphonic, ethanesulphonic, p-toluenesulphonic, benzenesulphonic or isethionic acid.

7. A process according to any one of the preceding claims, wherein L is $C_{1-4}$ alkoxy.

8. A process for the preparation of 4-amino-2-(4-n-propylpiperazin-1-yl)-5-(2,3,5-trichlorophenyl)pyrimidine or an acid addition salt thereof, which process comprises reacting a compound of formula (III):

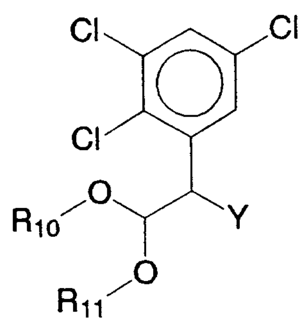

(III)

wherein Y is cyano and $R_{10}$ and $R_{11}$ are both alkyl or together form a group $-(C(R)_2)_n-$ where R is hydrogen or alkyl and n is an integer from 2 to 4, with the compound of formula (II) defined in claim 1 or a salt thereof; and isolating the thus formed 4-amino-2-(4-n-propylpiperazin-1-yl)-5-(2,3,5-trichlorophenyl)pyrimidine as the free base or as an acid addition salt thereof.

9. A process according to claim 8, wherein the said reaction is carried out in a non-aqueous solvent under reflux.

10. A process according to claim 9, wherein the solvent is ethanol.

11. A process according to any one of claims 8 to 10, wherein the compound of formula (II) is used in the form of the hydroiodide salt and the said reaction is carried out in the presence of a base.

12. A process according to claim 11, wherein the base is sodium ethoxide.

13. A process according to any one of claims 8 to 12, wherein the 4-amino-2-(4-n-propylpiperazin-1-yl)-5-(2,3,5-trichlorophenyl)pyrimidine is isolated as a salt formed from hydrochloric, hydrobromic, sulphuric, citric, tartaric, phosphoric, lactic, pyruvic, acetic, succinic, fumaric, maleic, oxaloacetic, methanesulphonic, ethanesulphonic, p-toluenesulphonic, benzenesulphonic or isethionic acid.

**Patentansprüche**

**Patentansprüche für folgende Vertragsstaaten : AT, BE, CH, DE, DK, FR, GB, IT, LI, LU, NL, SE**

1. 4-Amino-2-(4-n-propylpiperazin-1-yl)-5-(2,3,5-trichlorphenyl)pyrimidin und Säureadditionssalze davon.

2. Salz nach Anspruch 1, das ein pharmazeutisch verträgliches Säureadditionssalz ist.

3. Salz nach Anspruch 1, das ein Salz, gebildet aus Salzsäure, Bromwasserstoff, Schwefelsäure, Zitronensäure, Weinsäure, Phosphorsäure, Milchsäure, Traubensäure, Essigsäure, Succinsäure, Fumarsäure, Maleinsäure, Oxalessigsäure, Methansulfonsäure, Ethansulfonsäure, p-Toluolsulfonsäure, Benzolsulfonsäure oder Isethionsäure ist.

4. Salz nach Anspruch 1, das ein 4-Amino-2-(4-n-propylpiperazin-1-yl)-5-(2,3,5-trichlorphenyl)pyrimidindimesylat ist.

5. Verfahren zur Herstellung von 4-Amino-2-(4-n-propylpiperazin-1-yl)-5-(2,3,5-trichlorphenyl)pyrimidin oder einem Säureadditionssalz davon, welches umfaßt das Umsetzen einer Verbindung der Formel (I):

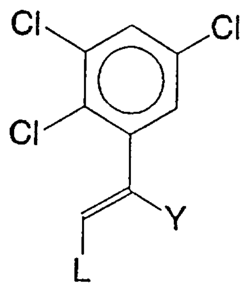

(I)

worin L eine Austrittsgruppe ist und Y ist Cyano, mit der Verbindung der Formel (II):

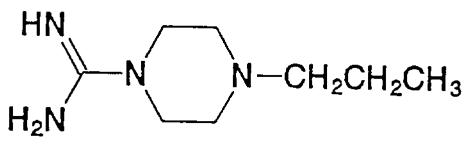

(II)

oder einem Salz davon; und Isolieren des so gebildeten 4-Amino-2-(4-n-propylpiperazin-1-yl)-5-(2,3,5-trichlorphenyl)pyrimidins als freie Base oder als ein Säureadditionssalz davon.

6. Verfahren nach Anspruch 5, worin L $C_{1-4}$-Alkoxy ist.

7. Verfahren zur Herstellung von 4-Amino-2-(4-n-propylpiperazin-1-yl)-5-(2,3,5-trichlorphenyl)pyrimidin oder einem Säureadditionssalz davon, welches umfaßt das Umsetzen einer Verbindung der Formel (III):

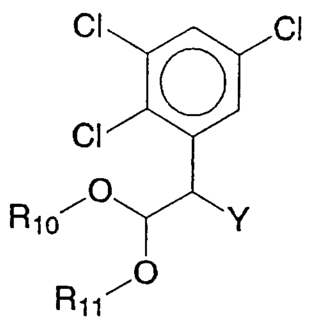

(III)

worin Y Cyano ist und $R_{10}$ und $R_{11}$ sind beide Alkyl oder bilden zusammen eine Gruppe $-(C(R)_2)_n-$ worin R Wasserstoff oder Alkyl ist, und n ist eine ganze Zahl von 2 bis 4, mit der Verbindung der Formel (II) gemäß Anspruch 5 oder ein Salz davon; und Isolieren des so gebildeten 4-Amino-2-(4-n-propylpiperazin-1-yl)-5-(2,3,5-trichlorphenyl)pyrimidins als freie Base oder als ein Säureadditionssalz davon.

8. Pharmazeutische Formulierung, enthaltend 4-Amino-2-(4-n-propylpiperazin-1-yl)-5-(2,3,5-trichlorphenyl)pyrimidin oder ein pharmazeutisch verträgliches Säureadditionssalz davon, und einen oder mehrere pharmazeutisch verträgliche Träger dafür.

9. Pharmazeutische Formulierung nach Anspruch 8, die für die orale Verabreichung geeignet ist.

10. Pharmazeutische Formulierung nach Anspruch 9, die in Form von Kapseln oder Tabletten vorliegt, die jeweils eine bestimmte Menge an 4-Amino-2-(4-n-propylpiperazin-1-yl)-5-(2,3,5-trichlorphenyl)pyrimidin oder ein pharmazeutisch annehmbares Säureadditionssalz davon enthält.

11. Pharmazeutische Formulierung nach Anspruch 8, die für die parenterale Verabreichung geeignet ist.

12. Pharmazeutische Formulierung nach Anspruch 11, die in Einheitsdosis-Behältern angeboten wird.

13. 4-Amino-2-(4-n-propylpiperazin-1-yl)-5-(2,3,5-trichlorphenyl)pyrimidin oder ein pharmazeutisch verträgliches Säureadditionssalz davon zur Verwendung in der Behandlung oder Prophylaxe eines Säugers oder einer neurodegenerativen oder anderen neurologischen Fehlfunktion des Zentralnervensystems, wobei die Ätiologie die überschüssige Freisetzung von Neurotransmitter-Glutamat umfaßt.

14. Verbindung nach Anspruch 13, worin die Fehlfunktion eine zerebrale ischämische Schädigung ist.

15. Verbindung nach Anspruch 14, worin die Schädigung durch einen Schlaganfall verursacht wurde.

16. Verbindung nach Anspruch 13, worin die Fehlfunktion eine physikalische Verletzung oder ein Trauma des Rückenmarks oder Gehirns ist.

**Patentansprüche für folgende Vertragsstaaten : ES, GR**

1. Verfahren zur Herstellung von 4-Amino-2-(4-n-propylpiperazin-1-yl)-5-(2,3,5-trichlorphenyl)pyrimidin oder ein Säureadditionssalz davon, welches umfaßt das Umsetzen einer Verbindung der Formel (I):

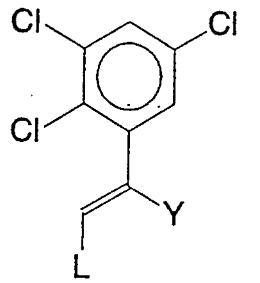

(I)

worin L eine Austrittsgruppe ist und Y ist Cyano, mit der Verbindung der Formel (II):

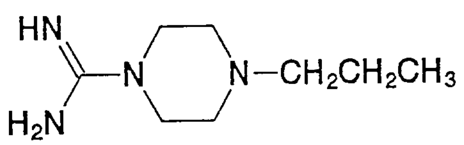

(II)

oder einem Salz davon; und Isolieren des so gebildeten 4-Amino-2-(4-n-propylpiperazin-1-yl)-5-(2,3,5-trichlorphenyl)pyrimidins als freie Base oder als ein Säureadditionssalz davon.

2. Verfahren nach Anspruch 1, worin die Reaktion durchgeführt wird in einem nichtwäßrigen Lösungsmittel bei 50 bis 110°C.

3. Verfahren nach Anspruch 2, worin das Lösungsmittel Ethanol ist.

4. Verfahren nach einem der vorhergehenden Ansprüche, worin die Verbindung der Formel (II) in Form des Hydrojodidsalzes verwendet wird, und die Reaktion unter Rückfluß in Gegenwart einer Base durchgeführt wird.

5. Verfahren nach Anspruch 4, worin die Base Natriumethoxid ist.

6. Verfahren nach einem der vorhergehenden Ansprüche, worin das 4-Amino-2-(4-n-propylpiperazin-1-yl)-5-(2,3,5-trichlorphenyl)pyrimidin als ein Salz, gebildet aus Salzsäure, Bromwasserstoff, Schwefelsäure, Zitronensäure, Weinsäure, Phosphorsäure, Milchsäure, Traubensäure, Essigsäure, Succinsäure, Fumarsäure, Maleinsäure, Oxalessigsäure, Methansulfonsäure, Ethansulfonsäure, p-Toluolsulfonsäure, Benzolsulfonsäure oder Isethionsäure isoliert wird.

7. Verfahren nach einem der vorhergehenden Ansprüche, worin L $C_{1-4}$-Alkoxy ist.

8. Verfahren zur Herstellung von 4-Amino-2-(4-n-propylpiperazin-1-yl)-5-(2,3,5-trichlorphenyl)pyrimidin oder einem Säureadditionssalz davon, welches umfaßt das Umsetzen einer Verbindung der Formel (III):

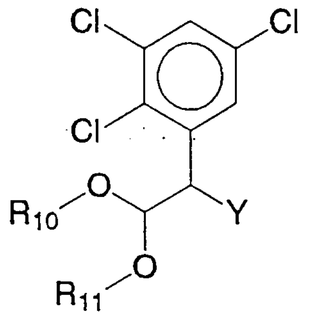

(III)

worin Y Cyano ist und $R_{10}$ und $R_{11}$ sind beide Alkyl oder bilden zusammen eine Gruppe $-(C(R)_2)_n-$ worin R Wasserstoff oder Alkyl ist, und n ist eine ganze Zahl von 2 bis 4, mit der Verbindung der Formel (II) gemäß Anspruch 5 oder ein Salz davon; und Isolieren des so gebildeten 4-Amino-2-(4-n-propylpiperazin-1-yl)-5-(2,3,5-trichlorphenyl)pyrimidins als freie Base oder als ein Säureadditionssalz davon.

9. Verfahren nach Anspruch 8, worin die Reaktion in einem nichtwäßrigen Lösungsmittel unter Rückfluß durchgeführt wird.

10. Verfahren nach Anspruch 9, worin das Lösungsmittel Ethanol ist.

11. Verfahren nach einem der vorhergehenden Ansprüche 8 bis 10, worin die Verbindung der Formel (II) in Form eines Hydrojodidsalzes verwendet wird, und die Reaktion in Gegenwart einer Base durchgeführt wird.

12. Verfahren nach Anspruch 11, worin die Base Natriumhydroxid ist.

13. Verfahren nach einem der vorhergehenden Ansprüche 8 bis 12, worin das 4-Amino-2-(4-n-propylpiperazin-1-yl)-5-(2,3,5-trichlorphenyl)pyrimidin als Salz, gebildet aus Salzsäure, Bromwasserstoff, Schwefelsäure, Zitronensäure, Weinsäure, Phosphorsäure, Milchsäure, Traubensäure, Essigsäure, Succinsäure, Fumarsäure, Maleinsäure, Oxalessigsäure, Methansulfonsäure, Ethansulfonsäure, p-Toluolsulfonsäure, Benzolsulfonsäure oder Isethionsäure isoliert wird.

**Revendications**

**Revendications pour les Etats contractants suivants : AT, BE, CH, DE, DK, FR, GB, IT, LI, LU, NL, SE**

1. 4-amino-2-(4-n-propylpipérazine-1-yl)-5-(2,3,5-trichlorophényl)pyrimidine et ses sels d'addition avec un acide.

2. Sel selon la revendication 1, qui est un sel d'addition avec un acide, acceptable d'un point de vue pharmaceutique.

3. Sel selon la revendication 1, qui est un sel formé à partir de l'acide chlorhydrique, bromhydrique, sulfurique, citrique, tartrique, phosphorique, lactique, pyruvique, acétique, succinique, fumarique, maléique, oxaloacétique, méthanesulfonique, éthanesulfonique, p-toluènesulfonique, benzènesulfonique ou iséthionique.

4. Sel selon la revendication 1, qui est le dimésylate de 4-amino-2-(4-n-propylpipérazine-1-yl)-5-(2,3,5-trichlorophényl)pyrimidine.

**5.** Procédé pour la préparation de la 4-amino-2-(4-n-propylpipérazine-1-yl)-5-(2,3,5-trichlorophényl)pyrimidine ou d'un de ses sels d'addition avec un acide, lequel procédé consiste à faire réagir un composé de formule (I) :

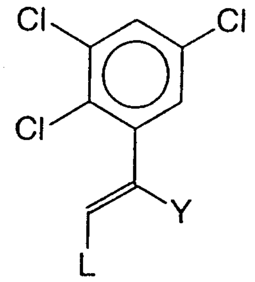

(I)

dans laquelle L est un groupe éliminable et Y est le radical cyano, avec le composé de formule (II) :

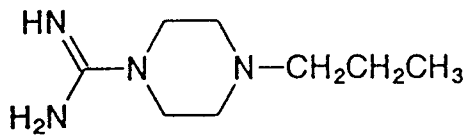

(II)

ou l'un de ses sels ; et à isoler la 4-amino-2-(4-n-propylpipérazine-1-yl)-5-(2,3,5-trichlorophényl)pyrimidine ainsi formée, sous forme de la base libre ou d'un de sels d'addition avec un acide.

**6.** Procédé selon la revendication 5, dans lequel L est un groupe alcoxy en $C_{1-4}$.

**7.** Procédé pour préparer la 4-amino-2-(4-n-propylpipérazine-1-yl)-5-(2,3,5-trichlorophényl)pyrimidine ou l'un de ses sels d'addition avec un acide, lequel procédé consiste à faire réagir un composé de formule (III)

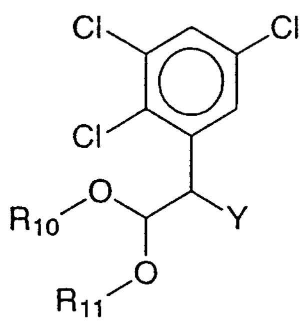

dans laquelle Y est le radical cyano, et $R_{10}$ et $R_{11}$ sont tous les deux des groupes alkyle ou forment ensemble un groupe $-C(R)_2)_n-$ où R est un hydrogène ou un radical alkyle et n est un entier de 2 à 4, avec le composé de formule (II) défini dans la revendication 5 ou l'un de ses sels ; et à isoler la 4-amino-2-(4-n-propylpipérazine-1-yl)-5-(2,3,5-trichlorophényl)pyrimidine ainsi formée, sous forme de la base libre ou d'un de ses sels d'addition avec un acide.

**8.** Formulation pharmaceutique comprenant de la 4-amino-2-(4-n-propylpipérazine-1-yl)-5-(2,3,5-trichlorophényl)pyrimidine ou l'un de ses sels d'addition avec un acide, acceptable d'un point de vue pharmaceutique, et un ou plusieurs excipients pour ce dernier, acceptables d'un point de vue pharmaceutique.

**9.** Formulation pharmaceutique selon la revendication 8, qui convient à une administration orale.

**10.** Formulation pharmaceutique selon la revendication 9, qui se présente sous forme de gélules ou de comprimés, contenant chacun une quantité prédéterminée de 4-amino-2-(4-n-propylpipérazine-1-yl)-5-(2,3,5-trichlorophényl)pyrimidine ou de ses sels d'addition avec un acide, acceptable d'un point de vue pharmaceutique.

**11.** Formulation pharmaceutique selon la revendication 8, qui convient à une administration parentérale.

**12.** Formulation pharmaceutique selon la revendication 11, qui est présentée en conditionnements unitaires.

**13.** 4-amino-2-(4-n-propylpipérazine-1-yl)-5-(2,3,5-trichlorophényl)pyrimidine, ou l'un de ses sels d'addition avec un acide, acceptable d'un point de vue pharmaceutique, pour utilisation dans le traitement ou la prophylaxie, chez un mammifère, d'un trouble neurodégénératif ou d'un autre trouble neurologique du système nerveux central, dont l'étiologie comprend la libération en excès du neurotransmetteur glutamate.

**14.** Composé selon la revendication 13, dans lequel le trouble est un dommage ischémique cérébral.

**15.** Composé selon la revendication 14, dans lequel le dommage a été provoqué par un accident cérébral vasculaire.

**16.** Composé selon la revendication 13, dans lequel le trouble est une blessure ou un traumatisme physiques de la moelle épinière ou du cerveau.

**Revendications pour les Etats contractants suivants : ES, GR**

**1.** Procédé pour la préparation de la 4-amino-2-(4-n-propylpipérazine-1-yl)-5-(2,3,5-trichlorophényl)-pyrimidine ou d'un de ses sels d'addition avec un acide, lequel procédé consiste à faire réagir un composé de formule (I) :

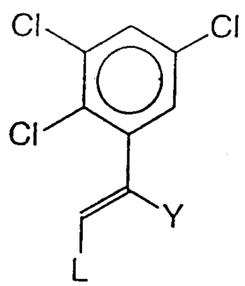

(I)

dans laquelle L est un groupe éliminable et Y est le radical cyano, avec le composé de formule (II) :

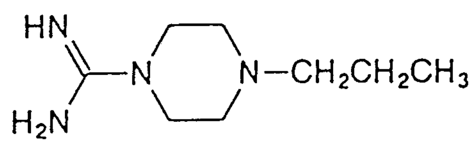

(II)

ou l'un de ses sels ; et à isoler la 4-amino-2-(4-n-propyl-pipérazine-1-yl)-5-(2,3,5-trichlorophényl)-pyrimidine ainsi formée, sous forme de la base libre ou d'un de sels d'addition avec un acide.

**2.** Procédé selon la revendication 1, dans lequel ladite réaction est mise en oeuvre dans un solvant non aqueux à une température de 50 à 110°C.

**3.** Procédé selon la revendication 2, dans lequel le solvant est l'éthanol.

**4.** Procédé selon l'une quelconque des revendications précédentes, dans lequel le composé de formule (II) est utilisé sous forme du sel iodhydrate, et ladite réaction est mise en oeuvre au reflux en présence d'une base.

**5.** Procédé selon la revendication 4, dans lequel la base est l'éthylate de sodium.

**6.** Procédé selon l'une quelconque des revendications précédentes, dans lequel la 4-amino-2-(4-n-propyl-pipérazine-1-yl)-5-(2,3,5-trichlorophényl)-pyrimidine est isolée sous forme d'un sel formé à partir de l'acide chlorhydrique, bromhydrique, sulfurique, citrique, tartrique, phosphorique, lactique, pyruvique, acétique, succinique, fumarique,

maléique, oxaloacétique, méthane-sulfonique, éthanesulfonique, p-toluènesulfonique, benzènesulfonique ou iséthionique.

7. Procédé selon l'une quelconque des revendications précédentes, dans lequel L est un groupe alcoxy en $C_{1-4}$.

8. Procédé pour préparer la 4-amino-2-(4-n-propyl-pipérazine-1-yl)-5-(2,3,5-trichlorophényl)-pyrimidine ou l'un de ses sels d'addition avec un acide, lequel procédé consiste à faire réagir un composé de formule (III)

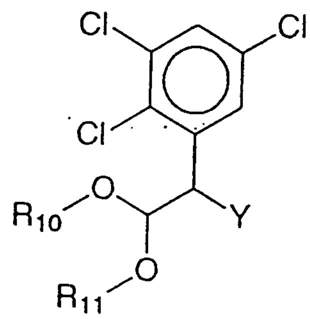

dans laquelle Y est le radical cyano, et $R_{10}$ et $R_{11}$ sont tous les deux des groupes alkyle ou forment ensemble un groupe $-C(R)_2)_n-$ où R est un hydrogène ou un radical alkyle et n est un entier de 2 à 4, avec le composé de formule (II) défini dans la revendication 5 ou l'un de ses sels ; et à isoler la 4-amino-2-(4-n-propylpipérazine-1-yl)-5-(2,3,5-trichlorophényl)-pyrimidine ainsi formée, sous forme de la base libre ou d'un de ses sels d'addition avec un acide.

9. Procédé selon la revendication 8, dans lequel ladite réaction est mise en oeuvre dans un solvant non aqueux au reflux.

10. Procédé selon la revendication 9, dans lequel le solvant est l'éthanol.

11. Procédé selon l'une quelconque des revendications 8 à 10, dans lequel le composé de formule (II) est utilisé sous forme du sel d'iodhydrate, et ladite réaction est mise en oeuvre en présence d'une base.

12. Procédé selon la revendication 11, dans lequel la base est l'éthylate de sodium.

13. Procédé selon l'une quelconque des revendications 8 à 12, dans lequel la 4-amino-2-(4-n-propyl-pipérazine-1-yl)-5-(2,3,5-trichlorophényl)-pyrimidine est isolée sous forme d'un sel formé à partir de l'acide chlorhydrique, bromhydrique, sulfurique, citrique, tartrique, phosphorique, lactique, pyruvique, acétique, succinique, fumarique, maléique, oxaloacétique, méthane-sulfonique, éthanesulfonique, p-toluènesulfonique, benzènesulfonique ou iséthionique.